(19) **Europäisches Patentamt / European Patent Office / Office européen des brevets**

(11) **EP 4 026 827 B1**

(12) **EUROPEAN PATENT SPECIFICATION**

(45) Date of publication and mention
of the grant of the patent:
**02.10.2024 Bulletin 2024/40**

(21) Application number: **20861364.6**

(22) Date of filing: **03.09.2020**

(51) International Patent Classification (IPC):
*C07C 319/28* (2006.01)    *C07C 323/52* (2006.01)
*A23K 20/105* (2016.01)    *A23K 20/20* (2016.01)

(52) Cooperative Patent Classification (CPC):
(C-Sets available)
**A23K 20/105; A23K 20/20; C07C 319/28**    (Cont.)

(86) International application number:
**PCT/CN2020/113133**

(87) International publication number:
**WO 2021/043188 (11.03.2021 Gazette 2021/10)**

(54) **MIXTURE CONTAINING METHIONINE HYDROXY ANALOG AND OLIGOMER THEREOF, AND PREPARATION METHOD THEREFOR**

MISCHUNG MIT METHIONIN-HYDROXY-ANALOGON UND OLIGOMER DAVON SOWIE VERFAHREN ZU IHRER HERSTELLUNG

MÉLANGE CONTENANT UN ANALOGUE D'HYDROXYMÉTHIONINE, UN OLIGOMÈRE DE CELUI-CI, ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2019 CN 201910830802**

(43) Date of publication of application:
**13.07.2022 Bulletin 2022/28**

(73) Proprietor: **Bluestar Adisseo Nanjing Co., Ltd.**
**Nanjing, Jiangsu 211500 (CN)**

(72) Inventors:
• **GUIDAL, Valentin**
  **Nanjing, Jiangsu 211500 (CN)**
• **NIU, Lei**
  **Nanjing, Jiangsu 211500 (CN)**
• **SANCHEZ, Francisco**
  **Nanjing, Jiangsu 211500 (CN)**

(74) Representative: **Germain Maureau**
**12, rue Boileau**
**69006 Lyon (FR)**

(56) References cited:
CN-A- 1 186 068    CN-A- 1 315 939
CN-A- 105 130 861   CN-A- 109 232 336
CN-A- 109 232 336   CN-A- 109 232 338
CN-A- 110 483 348

EP 4 026 827 B1

(52) Cooperative Patent Classification (CPC): (Cont.)

C-Sets
**C07C 319/28, C07C 323/58**

**Description**

TECHNICAL FIELD

The present disclosure relates to a mixture containing a methionine hydroxy analog and an oligomer thereof, and a preparation method thereof.

BACKGROUND ART

Methionine hydroxy analogs, such as 2-hydroxy-4-(methylthio)butanoate (HMTBA), are in the liquid form and are organic acids with strong acidity. Liquid hydroxymethionine can be prepared by cyanohydrin hydrolysis, in which methylthiopropanal is reacted with hydrocyanic acid to prepare 2-hydroxy-4-(methylthio)butyronitrile (HMTBN). Under the action of sulfuric acid, 2-hydroxy-4-(methylthio)butyronitrile is hydrated into 2-hydroxy-4-(methylthio)butanamide, and then, under the action of sulfuric acid, 2-hydroxy-4-(methylthio)butanamide is hydrolyzed into a hydrolysis product containing liquid hydroxymethionine, ammonium sulfate, and ammonium bisulfate. It is necessary to remove ammonium sulfate and ammonium bisulfate from the hydrolysis product to obtain high-purity liquid hydroxymethionine.
In order to facilitate transportation, especially at low temperature in winter, and subsequent processing, it is desirable to reduce the viscosity of liquid hydroxymethionine.
CN109232336A and CN 1186068A disclose mixtures comprising a methionine hydroxy analog and an oligomer thereof.

SUMMARY

The inventors have found that by removing salts, particularly ammonium sulfate and ammonium bisulfate, from a methionine hydroxy analog to a certain extent, the equilibrium between a monomer and an oligomer of the methionine hydroxy analog can be adjusted, and changes in the amount and the ratio unexpectedly affect the viscosity of the product.
In one aspect, the present disclosure provides a mixture, containing:

86-89 wt% of a methionine hydroxy analog and an oligomer thereof, more than 99.95 wt% being a monomer, dimer, and trimer, and the ratio of monomer: dimer: trimer by weight being 77-80: 18.5-19.5: 3-4;

9.2-13.5 wt% of water; and
less than 0.5 wt% of ammonium sulfate and ammonium bisulfate; and

the viscosity of the mixture at 10°C being lower than 200 mPa·s.

In another aspect, the present disclosure provides a method for processing a mixture containing a methionine hydroxy analog and an oligomer thereof, including the following steps:

S1: placing the mixture in contact with an ion exchange resin;
S2: placing the mixture in contact with another ion exchange resin;
S3: washing the ion exchange resin of step S1 with water to obtain an aqueous eluent, and diluting a mixture containing a methionine hydroxy analog and an oligomer thereof with the aqueous eluent before step S1, and/or
washing the ion exchange resin of step S2 with water to obtain an aqueous eluent, and diluting a mixture containing a methionine hydroxy analog and an oligomer thereof with the aqueous eluent before step S1; and
S4: regenerating the ion exchange resins.

The above and other features and advantages of the present disclosure will become apparent from the following detailed description of the embodiments and the appended claims.

BRIEF DESCRIPTION OF THE DRAWINGS

The present disclosure will be further described with reference to the drawings:

Fig. 1 is a flowchart of a method according to an embodiment; and
Fig. 2 is a schematic diagram of a preparation process of HMTBA in which a desorption solution and/or a cleaning solution obtained by the resin processing are recirculated.

DETAILED DESCRIPTION OF THE INVENTION

Unless otherwise specified, the following definitions are provided to illustrate and define the meaning and scope of various terms used in the present disclosure.

Herein, the term "*mixture*" refers to a combination of two or more different substances.

The term "*AS*" refers to ammonium sulfate with a molecular formula of $(NH_4)_2SO_4$.

The term "*BAS*" refers to ammonium bisulfate with a molecular formula of $NH_4HSO_4$.

The term "*HMTBA*" refers to 2-hydroxy-4-(methylthio)butanoate, also known as *methionine hydroxy analog* or *liquid methionine.*

The term "*ATS*" refers to an ammonium salt of 2-hydroxy-4-(methylthio)butanoate (HMTBA).

The term "*MTN*" refers to methionine.

The term "*BV*" refers to bed volume.

The term "*TOS*" refers to total organic sulfur. It is a measure of the content of sulfur-containing organic compounds in a sample.

The term "*HPLC*" refers to high performance liquid chromatography.

The term "*DMW*" refers to demineralized water.

The term "*MMP*" refers to methylthiopropanal.

Unless otherwise specified, amounts and percentages are based on weight.

The present disclosure provides a mixture containing a methionine hydroxy analog and an oligomer thereof, and a preparation method thereof. By reducing the content of ammonium sulfate and ammonium bisulfate in the mixture to be less than 0.5 wt%, the content of water in the mixture is increased, and the total amount of a monomer, a dimer, and a trimer of the methionine hydroxy analog is increased to make the viscosity of the mixture at 10°C lower than 200 mPa·s.

As a cyanohydrin hydrolysis product, the mixture containing a methionine hydroxy analog as a main component may contain salts such as ammonium sulfate (AS), ammonium bisulfate (BAS), an ammonium salt of 2-hydroxy-4-(methylthio)butanoate (ATS), and methionine (MTN). The inventors have found that the salts are not only undesired impurities, but also factors affecting the viscosity of the mixture. AS and BAS can be removed from the mixture by the ion exchange resin processing, the ion exchange resin processing is combined to a preparation process to circulate an effluent, so that no waste water will be discharged from the whole process. Furthermore, the removed salts can be recycled, so no solid waste will be produced. Therefore, the process can effectively utilize resources and is environmentally-friendly.

In some embodiments, a mixture is obtained by cyanohydrin hydrolysis, in which the total amount of salts is 1.3-1.6 wt%, and AS and BAS are main components. For example, the total amount is 1.2-1.4 wt%, and the total amount of ATS and MTN is less than 0.2 wt%, particularly less than 0.1 wt%.

AS and BAS are removed to a certain extent by processing the mixture with ion exchange resins.

In some embodiments, the mixture is processed to obtain a mixture below, which includes:

86-89 wt% of a methionine hydroxy analog and an oligomer thereof, more than 99.95 wt% being a monomer, dimer, and trimer, and the ratio of monomer: dimer: trimer by weight being 77-80: 18.5-19.5: 3-4;

9.2-13.5 wt% of water; and
less than 0.5 wt% of ammonium sulfate and ammonium bisulfate; and

the viscosity of the mixture at 10°C being lower than 200 mPa·s.

In some embodiments, the ratio of monomer: dimer: trimer by weight is 77.4-78.5: 18.5-19.2: 3.2-3.6.

In view of the quality of the product, the monomer is appropriate. The relatively low content of salts and the relatively high content of water in the mixture break the equilibrium between the monomer and the oligomer and make the content of the monomer increase. The dimer and the trimer are less appropriate, and the viscosity of them is higher than the viscosity of the monomer, so the relatively high content of monomer helps reduce the viscosity of the mixture.

Particularly, the mixture contains 9.5 wt%, 10 wt%, 10.5 wt%, 11 wt%, 11.5 wt%, 12 wt%, 12.5 wt% or 13 wt% of water, and/or less than 0.4 wt% of ammonium sulfate and ammonium bisulfate. In some embodiments, the mixture contains less than 0.4 wt% of ammonium sulfate, and less than 0.1 wt% of ammonium bisulfate.

Because the content of salts is reduced, and the content of water is increased, the viscosity of the mixture can be reduced to be lower than 200 mPa·s at 10°C. Particularly, the viscosity at 10°C is lower than 170 mPa·s. In some embodiments, the viscosity at 0°C is lower than 340 mPa·s.

The mixture of the present disclosure can be used as a feed additive. For example, the mixture is used in a nutritional composition for animals or humans, and the nutritional composition can be dried as a solid for subsequent use. For example, the mixture can be spray-dried, and the low content of salts and the appropriate viscosity can solve some problems in the spray drying process, such as precipitation and difficult processing.

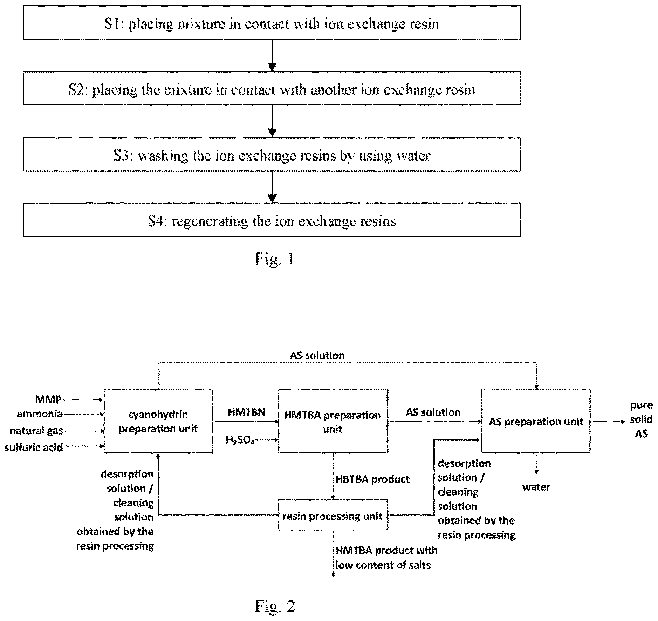

As shown in Fig. 1, the present disclosure provides a method for processing a mixture containing a methionine hydroxy analog and an oligomer thereof, including the following steps:

S1: placing the mixture in contact with an ion exchange resin;
S2: placing the mixture in contact with another ion exchange resin;
S3: washing the ion exchange resin of step S1 with water to obtain an aqueous eluent, and diluting a mixture containing a methionine hydroxy analog and an oligomer thereof with the aqueous eluent before S1;
and/or
washing the ion exchange resin of step S2 with water to obtain an aqueous eluent, and diluting a mixture containing a methionine hydroxy analog and an oligomer thereof with the aqueous eluent before S1; and
S4: regenerating the ion exchange resins.

In some embodiments, steps S1 and S2 respectively include removing $NH_4^+$ with a cation exchange resin, and removing $SO_4^{2-}$ an anion exchange resin. For example, step S1 includes:

S11: placing the mixture in contact with a cation exchange resin, particularly in contact with a strong-acid cation exchange resin;
and step S2 includes:

S21: the mixture is placed in contact with an anion exchange resin, particularly in contact with a weak-base anion exchange resin;
or
step S1 includes:

S12: placing the mixture in contact with an anion exchange resin, particularly in contact with a weak-base anion exchange resin;
and step S2 includes:
S22: placing the mixture in contact with a cation exchange resin, particularly in contact with a strong-acid cation exchange resin.

In some embodiments, before step S1, the mixture containing a methionine hydroxy analog and an oligomer thereof contains less than 12 wt% of water, particularly less than 11 wt% of water; and more than 1 wt% of ammonium sulfate and ammonium bisulfate, particularly more than 1.2 wt% of ammonium sulfate and ammonium bisulfate, and more particularly 1.3-1.6 wt% of ammonium sulfate and ammonium bisulfate. In some embodiments, before step S1, the viscosity of the mixture containing a methionine hydroxy analog and an oligomer thereof at 10°C is higher than 350 mPa·s.

The cation exchange resin may be a strong-acid cation exchange resin, for example, a strong-acid cation exchange resin having sulfonic groups as exchange groups, or a weak-acid cation exchange resin, for example, a weak-acid cation exchange resin having carboxy groups as exchange groups. Particularly, the cation exchange resin is a strong-acid cation exchange resin. In some embodiments, a skeleton structure of the cation exchange resin is a styrene-divinylbenzene copolymer, ion exchange groups are sulfonic groups, sizes of 95% or above of particles are in a range of 0.315-1.25 mm, an exchange capacity is equal to or higher than 1.9 mmol/mL, a specific surface area is equal to or greater than 50 $m^2$/g, and the content of water is 50-60%. The anion exchange resin may be a weak-base anion exchange resin, for example, a weak-base anion exchange resin having primary, secondary, and tertiary amines as exchange groups, or a strong-base anion exchange resin, for example, a strong-base anion exchange resin having quaternary ammonium groups as exchange groups. Particularly, the anion exchange resin is a weak-base anion exchange resin, for example, a skeleton structure of the anion exchange resin is a methacrylate polymer, ion exchange groups are tertiary amines, sizes of 95% or above of particles are in a range of 0.315-1.25 mm, an exchange capacity is equal to or higher than 1.6 mmol/mL, a specific surface area is equal to or greater than 50 $m^2$/g, and the content of water is 55-65%. Before the first use, the resins are activated, for example, the cation resin is activated with 10 wt% sulfuric acid, and the anion resins is activated with 10 wt% ammonia water, so that the resins are saturated with $H^+$ or $OH^-$.

At the ion exchange step, a feed liquid passes through the resins to remove salts (ammonium ions or sulfate ions) from the feed liquid. Parameters, such as the feed temperature, a feed rate (in BV/h), and the volume (in BV) of a feed liquid processed with 1 BV of resin, can affect an ion exchange result.

With regarding to the viscosity of a mixture to be processed, the feed temperature is higher than 45°C. However, if the temperature is too high, the service life of the resin will be shortened. In some embodiments, steps S1 and S2 are performed at the temperature of 45-55°C, and particularly performed at 50°C.

A rate of a feed liquid entering a resin column may affect a desalting efficiency. In some embodiments, steps S1 and S2 are performed at a rate of 0.8-2.5 BV/h, particularly performed at a rate of 1-2 BV/h.

A capacity (C, in mL/g) of a resin refers to an amount of $NH_4^+$ or $SO_4^{2-}$ that can be exchanged per unit volume of the resin. It is determined by measuring $NH_4^+$% and $SO_4^{2-}$% at the inlet and outlet, and measuring the weight of the feed liquid passing through the resin in saturated BV. For example, a capacity of the cation exchange resin is calculated by the following formula.

$$C_{cation}(ml/g) = \frac{\text{volume of resin}}{\text{weight of feed liquid} \times \text{inlet } NH_4\% - \text{weight of effluent} \times \text{outlet } NH_4\%}$$

A capacity of the anion exchange resin is calculated by the following formula.

$$C_{anion}(ml/g) = \frac{\text{volume of resin}}{\text{weight of feed liquid} \times \text{inlet } SO_4\% - \text{weight of effluent} \times \text{outlet } SO_4\%}$$

The capacity of the resin, and the number of ions to be removed with the resin are used to determine BV of the resin required for the processing. BV of the used cation exchange resin and anion exchange resin may be different.

In some embodiment, for the cation exchange resin, the resin becomes saturated when processing 10 BV, such as 8 BV, of feed liquid, and the anion exchange resin becomes saturated when processing 10 BV, such as 8 or 7 BV, of feed liquid, which is the maximum volume of a mixture processed with 1 BV of resin. Otherwise, the content of salts at the outlet of the ion exchange resin is unsatisfactory. Therefore, at each of steps S1 and S2, 1 BV of ion exchange resin is used to process at most 10 BV, particularly at most 8 BV, of mixture containing a methionine hydroxy analog and an oligomer thereof.

In some embodiments, after step S2, the concentration of $NH_4^+$ in the mixture containing a methionine hydroxy analog and an oligomer thereof is 0.02-0.1%, such as 0.03-0.08%, for example, about 0.04%, the concentration of $SO_4^{2-}$ is 0.02-0.2%, such as 0.05-0.12%, for example, about 0.07%, and/or the pH at 25°C is 0.3-2.5, such as 0.5-1.5, for example, about 0.7, about 0.8, about 0.9 or about 1.0.

At steps S1 and S2, after becoming saturated, the ion exchange resins are washed with water, such as DMW, to remove the remaining TOS on the resins. At step S3, the ion exchange resin of each of steps S1 and S2 is washed with water, such as DMW, to obtain an aqueous eluent, and the aqueous eluent is used to dilute a mixture containing a methionine hydroxy analog and an oligomer thereof before step S1. An effluent obtained by the washing of step S3 is recycled into the process, so that no waste water will be produced.

In some embodiments, the water used at step S3 is not heated before flowing into the resin column because there is no special requirement for the temperature of this step.

The volume of water used for washing is a factor affecting the overall efficiency of the method. If the volume of water used at the washing step is insufficient, a certain amount of TOS will remain on the resin, and this amount of TOS will enter a regeneration solution at step S4 to cause contamination and product loss. In some embodiments, at step S3, at least 2 BV, for example, at least 3 BV, of water is used to wash the ion exchange resin of each of steps S1 and S2.

In some embodiments, step S3 is performed at a rate of 1-3 BV/h, particularly performed at a rate of 1.5-2.5 BV/h.

After the washing of step S3, the ion exchange resins are regenerated and used for the next processing cycle, in which a generation solution passes through the resin to remove $NH_4^+$ and $SO_4^{2-}$, and the resin is saturated again with $H^+$ and $OH^-$. Regeneration of step S4 includes:

S41: the cation exchange resin is washed with an acid solution, such as a strong acid solution, particularly a sulfuric acid solution, to obtain a desorption solution, and the anion exchange resin is washed with a base solution, such as a weak base solution, particularly an ammonia-water solution, to obtain another desorption solution;
S42: the cation exchange resin and/or anion exchange resin are washed with water to obtain a cleaning solution; and
S43: the cation exchange resin and the anion exchange resin are washed with a mixture containing a methionine hydroxy analog and an oligomer thereof, particularly the mixture containing a methionine hydroxy analog and an oligomer thereof that is processed at steps S1 and S2.

In some embodiments, the desorption solution and/or the cleaning solution are recirculated in the preparation process of HMTBA.

Fig. 2 is a schematic diagram of a preparation process of HMTBA in which a desorption solution and/or a cleaning solution obtained by the resin processing are recirculated. A preparation system of HMTBA by cyanohydrin hydrolysis may include at least a cyanohydrin preparation unit, an HMTBA preparation unit, and an AS preparation unit.

In the cyanohydrin (HMTBN) preparation unit, MMP and hydrogen cyanide (HCN, prepared from ammonia and natural gas) are used to prepare cyanohydrin. A certain amount of sulfuric acid and water are further introduced into the unit. The desorption solution and/or the cleaning solution can be added into the unit to replace a certain amount of water. In the HMTBA preparation unit, HMTBN is used to prepare HMTBA under the action of $H_2SO_4$, and an effluent can be processed by the method of the present disclosure to obtain a mixture containing HMTBA and low content of salts. In the AS preparation unit, all effluents containing AS (at different AS% concentrations) from other units are processed, water is evaporated, and crystallization is performed to obtain solid pure AS serving as a by-product. A desorption solution and/or a cleaning solution containing AS can be delivered to the unit.

In some embodiments, the desorption solution and/or the cleaning solution can be used as a feed liquid in the AS preparation unit or used as feed water in the cyanohydrin hydrolysis unit.

In some embodiments, the concentration of the sulfuric acid solution is 5-20 wt%, particularly 8-12 wt%, and the concentration of the ammonia solution is 2-20 wt%, particularly 4-12 wt%. The sulfuric acid solution can be prepared from 98 wt% sulfuric acid, and the ammonia solution can be prepared from 32 wt% ammonia water or ammonia gas.

If the volume of the regeneration reagent used in the regeneration step is insufficient, an efficiency of the next ion exchange processing cycle will not be 100%. The degree of regeneration can be assessed by the following parameters, such as the pH or the content of $NH_4^+$ and/or $SO_4^{2-}$ at the regeneration outlet, and the volume of a solution (in BV) that passes through the resin to achieve the desired specification at the regeneration outlet.

In some embodiments, at step S41, the pH of an effluent from the cation exchange resin is lower than 2, and/or the concentration of $NH_4^+$ is less than 0.1%. The pH of an effluent from the anion exchange resin is greater than 10, and/or the concentration of $SO_4^{2-}$ is less than 0.05%.

In some embodiments, step S4 is performed at the temperature of 40°C to 55°C, and/or step S4 is performed at a rate of 1.5 BV/h to 2.5 BV/h, particularly performed at a rate of 1.8 BV/h to 2.2 BV/h.

In some embodiments, at step S41, at least 2 BV, particularly 2.5-3.5 BV, of above sulfuric acid solution is used, and/or at least 1.2 BV, particularly 1.5-2 BV, of above ammonia solution is used. In some embodiments, 3 BV of 10 wt% $H_2SO_4$ solution is used, and 1.5-2 BV of 10 wt% ammonia solution is used.

In some embodiments, 10 wt% of $H_2SO_4$ solution passes through the cation exchange resin until the pH of an effluent is smaller than 2 to regenerate and activate the resin, and 10 wt% of $NH_3 \cdot H_2O$ solution passes through the anion exchange resin until the pH of an effluent is greater than 10 to regenerate and activate the resin.

After the regeneration of step S41, the ammonia water or sulfuric acid solution remaining in the columns needs to be washed out to ensure effective ion exchange and avoid a small amount of acid/base in TOS. At step S42, water, such as DMW, passes through the resin columns to remove a regeneration reagent and make the pH of an effluent from the cation exchange resin be 3-4, and/or the pH of an effluent from the cation exchange resin be 9-10, and a cleaning solution is collected and recycled into the process, for example, used as feed water in the cyanohydrin hydrolysis unit. In some embodiments, at step S42, each resin is washed with at least 2 BV, particularly at least 3 BV, of water. For example, the cation exchange resin is washed with at least 2 BV, particularly at least 3 BV, of water, and the anion exchange resin is washed with at least 2 BV, particularly at least 3 BV, of water.

At step S43, the cation exchange resin and the anion exchange resin are washed with the mixture containing a methionine hydroxy analog and an oligomer thereof. Particularly, at step S43, the cation exchange resin and the anion exchange resin are washed with the mixture containing a methionine hydroxy analog and an oligomer thereof that is processed with resins, that is, the mixture is processed at steps S1 and S2, so that the columns are filled with a mixture containing HMTBA and low content of salts instead of water prior to the start of adsorption in a new processing cycle. It avoids dilution/contamination of the processed product with water.

In some embodiments, steps S1 to S4 are performed at the same time on at least two groups of ion exchange resins, and each group of ion exchange resins includes at least one cation exchange resin and at least one anion exchange resin. Particularly, the at least two groups of ion exchange resins do not undergo the same step at the same time. Much particularly, at least one group of ion exchange resins undergoes steps S 1 and S2, and at least one group of ion exchange resins undergoes step S4, so that the ion exchange processing (steps S 1 and S2) can be performed on at least one group of ion exchange resins at any time. Therefore, the processing can be performed in a continuous manner for consistent results.

AS and BAS in a mixture can be reduced to be less than 0.5 wt% by the method of the present disclosure. For 1 kg of removed AS, 2 kg of AS is produced. However, the removed salts and newly produced salts can be recirculated in the preparation process, can reach the solid AS preparation unit, and then can be processed into a solid AS by-product. Therefore, the method will not produce solid waste.

## *Example*

The following examples and comparative examples are provided to help understanding of the present disclosure, but should not be construed to limit the scope of the present disclosure. Unless otherwise specified, all parts, percentages, concentrations, etc. are based on weight. The following measuring methods and procedures are used for the evaluation of the following examples and comparative examples.

### Characterization method

Characterization methods will be described below. Unless otherwise specified, all measurement were performed at room temperature.

### Reagent and material

Unless otherwise specified, all reagents were analytically pure, water was distilled water or equivalently pure.

### The pH

The pH of a solution was directly measured by using a calibrated pH meter (InLab® 413 Combined Electrode, made by Mettler, or an equivalent) while the solution was vigorously stirred. The pH meter was calibrated before any measurement, and automatically corrected for temperature.

Buffer solutions with the pH of 2 and 4: product code 272581 and 272168, were made by Panreac, or equivalents.

### TOS

Total organic sulfur (TOS) was measured based on oxidization of thioether groups with a bromine oxidizer (generated in situ from a mixture of bromide/bromate ester and an acid medium) to sulfoxide. An equivalent point was determined by potentiometry.

### Titration medium

120 g of potassium bromide (product code 141489, made by PANREAC, or an equivalent) was dissolved in distilled water, 160 mL of 35% hydrochloric acid (product code 721019, made by PANREAC, or an equivalent) was added, and the volume of the system was made up to 1 L with distilled water.

Bromine: 0.1 N, product code 182000, made by PANREAC, or an equivalent.

### Device

METTLER DL 25, DL 40 or DL 53 titrator, or an equivalent, equipped with double-pin platinum electrodes (product code DM 142), combined with a polarization module DK 102 (used for METTLER DL 40 and DL 25).

### Procedure

About 0.2-0.3 g of acid phase to be analyzed was weighted out by using an analytical balance, and transferred into a titration beaker. About 50 mL of titration medium was added to titrate.

### Concentrations of AS and BAS

The content of AS and BAS was calculated based on measurement of sulfate ions and ammonium.

Ammonium was measured by potentiometric titration using a standard solution of sodium hydroxide (NaOH).

### Principle and reaction

The content of ammonium was determined by titration using a basic compound of known normality. The titration was performed in a non-aqueous medium to highlight the potential step due to the presence of bisulfate ions.

### Reagent and material

Sodium hydroxide: 1N, product code 181691, made by PANREAC, or product code SO0441, made by SCHARLAU, or an equivalent.

Isopropyl alcohol QP (technical pure)

*Device*

Titrator METTLER DL 25 or DL 53, or an equivalent.

Combined with a pH electrode used for a non-aqueous medium (DG 115 or an equivalent).

*Procedure*

About 1 g of sample was weighed out, and 30 mL of isopropyl alcohol was added. The system was titrated to an equivalence point of ammonium ions.

Sulfate ions were measured by potentiometric titration using a standard solution of lead perchlorate ($Pb(ClO_4)_2$).

*Principle and reaction*

The content of sulfate ions was determined by titration using a standard solution of lead perchlorate.

*Reagent and material*

$Pb(ClO_4)_2$ solution: 0.02 M ORION 948206

*Device*

Potentiometer (Titrando 905 Metrohm or an equivalent).

- ion selective electrode, specific Pb (ex: Metrohm n° 6- 0502-170).
- reference electrode

Saturated calomel electrode (ex: Tacussel n° XR 160 513 137) or Ag/AgCl (filled with tetraethylammonium perchlorate saturated with ethanol).

*Procedure*

About 1 g of sample was weighed out, and dissolved in 80 mL of acetone/water mixture (v/v=1.25/0.75). The system was titrated to an equivalence point of sulfate ions.

*Content of water*

The content of water was determined by normal Karl Fischer titration using a KARL-FISCHER reagent in an anhydrous solution of iodine and sulfurous acid anhydride. An equivalent point was determined by precise discoloration.

*Principle and reaction*

The determination was performed by titration using a KARL-FISCHER reagent in an anhydrous solution of iodine and sulfurous acid anhydride. In the presence of water, iodine oxidizes sulfurous acid anhydride, which was then reduced to iodide. At the equivalent point, the excess reagent caused the color to change from yellow to dark red. In order to better detect the end point, polarized platinum electrodes were used.

*Reagent and material*

Methanol used for measurement of content of water (low content of water), product code 171091, made by PANREAC, or a HYDRANAL solvent for volumetric Karl Fischer, product code 34800, made by RIEDEL DE HAËN.

KARL-FISCHER reagent solution (1 $cm^3$ = 5 mg of water), product code RE 0013, made by SCHARLAU, or a HYDRANAL titrant 5, made by RIEDEL DE HAËN, product code 34801, or product code 171574, made by PANREAC.

*Device*

Titrator METTLER DL 18 or DL 35/DL 31, equipped with double-pin platinum electrodes (DM 142 or an equivalent) and a polarization module DK 102 (only in DL 18).

*Procedure*

An analytical medium is neutralized in a titration beaker. Once the analytical medium is in the neutral condition, about 0.2 g of sample is added to the titration beaker. The titrator starts and titrates the system to the equivalence point; and the titrator stops automatically and gives a result based on water%.

*Content of monomer, dimer, and trimer in the mixture containing a methionine hydroxy analog and an oligomer thereof*

Monomers, dimers, and trimers of HMTBA were determined by HPLC (Agilent, HPLC 1260, chromatographic column: Nucleosil 100-5 C18 (250 × 4.6 mm), UV detector, eluent: acetonitrile/water, 0.8 mL/min, 30°C, 400 bar).

*Viscosity*

The viscosity was determined by a standard method using a CANNON-FENSKE capillary viscometer for transparent liquids, suitable for the viscosity range to be measured (300 series), standards ASTM D 445 and ISO 3104. The viscometer comes with a calibration certificate.

*Resin*

Resins used in the examples were provided by SUNRESIN, which are listed in details in Table 1 and Table 2.

Table 1 Cation exchange resin

| Appearance | Brown to gray opaque particles |
|---|---|
| Skeleton structure | Styrene-divinylbenzene copolymer |
| Particle size 0.315-1.25 mm | ≥95% |
| Exchange capacity | ≥1.9 mmol/ml |
| Content of water | 50-60% |
| Specific surface area | ≥50 $m^2$/g |
| Ion exchange group | Sulfonic groups |

Table 2 Anion exchange resin

| Appearance | White translucent particles |
|---|---|
| Skeleton structure | Methyl acrylate polymer |
| Particle size 0.315-1.25 mm | ≥95% |
| Exchange capacity | ≥1.6 mmol/ml |
| Content of water | 55-65% |
| Specific surface area | ≥50 $m^2$/g |
| Ion exchange group | Tertiary amines |

*Example 1: Capacity of resin*

*Column device*

The device is composed of two jacket type glass columns (one is used for a cation exchange resin, and the other is used for an anion exchange resin), a cation exchange resin washing system, an anion exchange resin washing system, and a demineralized water washing system. The jacket could be filled with water at the temperature required by the test. The columns were filled with a required amount (i.e. 1 bed volume (BV)) of resin. A mixture to be processed, a sulfuric acid solution, an ammonia solution or demineralized water passed through the columns via a peristaltic pump to be subjected to regeneration, ion exchange or washing. A feed rate of the column can be precisely controlled by the peristaltic pump.

*Procedure*

Parameters for ion exchange were fixed to 2 BV/h (1 BV = 150 ml) and 50°C. Tests on two capacities were performed, and the pH, TOS, $NH_4^+$, and $SO_4^{2-}$ at the outlet of the resin were monitored. 1,260 g of mixture containing a methionine hydroxy analog was fed into each volume in the system, and an initial effluent (0-0.5 BV) was discarded. Then, all effluents containing TOS were collected (after 0.5 BV, and 2 BV of cleaning water). A capacity of the resin was calculated by the following formula. Results are shown in Tables 3 and 4.

$$C_{cation}(ml/g) = \frac{volume\ of\ resin}{weight\ of\ feed\ liquid \times inlet\ NH_4\% - weight\ of\ effluent \times outlet\ NH_4\%}$$

$$C_{anion}(ml/g) = \frac{volume\ of\ resin}{weight\ of\ feed\ liquid \times inlet\ SO_4\% - weight\ of\ effluent \times outlet\ SO_4\%}$$

Table 3 Test on capacity of cation exchange resin

|  | TOS (%) | Water (%) | $SO_4^{2-}$ (%) | pH | $NH_4^+$ (%) | Weight (g) | TOS (g) | $NH_4^+$ (g) | Capacity (ml/g) |
|---|---|---|---|---|---|---|---|---|---|
| Feed liquid | 90.34 | 9.48 | 1.08 | 0.76 | 0.37 | 1260.00 | 1138.28 | 4.66 | 36.81 |
| Effluent | 77.05 | 23.90 | 0.94 | 0.39 | 0.04 | 1468.60 | 1131.56 | 0.59 | |
| Water for washing | 0.44 | | | | 0.00 | 286.00 | 1.26 | 0.00 | |

Table 4 Test on capacity of anion exchange resin

|  | TOS (%) | Water (%) | $SO_4^{2-}$ (%) | pH | $NH_4^+$ (%) | Weight (g) | TOS (g) | $SO_4^{2-}$ (g) | Capacity (ml/g) |
|---|---|---|---|---|---|---|---|---|---|
| Feed liquid | 90.34 | 9.48 | 1.08 | 0.76 | 0.37 | 1260.00 | 1138.28 | 13.61 | 16.57 |
| Effluent | 69.14 | 30.92 | 0.30 | 1.82 | 0.27 | 1518.40 | 1049.82 | 4.56 | |
| Water for washing | 0.15 | | 0.09 | | | 326.50 | 0.49 | 0.29 | |

*Example 2 Continuous ion exchange test*

1,000 g of mixture containing a methionine hydroxy analog was continuously fed into a system (for the cation exchange resin, 1 BV = 125 mL, and for the anion exchange resin, 1 BV = 150 mL), the mixture first passed through the cation exchange resin and then passed through the anion exchange resin at a flow rate of 2 BV/h and 50°C. Each BV of the sample was analyzed. Results are shown in Table 5 below.

Table 5 Results of Example 2

|  | 1 BV | 2 BV | 3 BV | 4 BV | 5 BV | 6 BV | 7 BV | 8 BV |
|---|---|---|---|---|---|---|---|---|
| pH | 1.130 | 0.850 | 0.860 | 0.870 | 0.960 | 1.020 | 0.870 | 0.680 |
| Water (%) | 21.21 | 11.30 | 10.01 | 9.65 | 9.51 | 9.24 | 9.19 | 9.15 |
| AS (%) | 0.183 | 0.215 | 0.234 | 0.280 | 0.396 | 0.583 | 0.949 | 1.160 |
| BAS (%) | 0.000 | 0.000 | 0.000 | 0.000 | 0.000 | 0.000 | 0.000 | 0.000 |
| AS (%) + BAS (%) | 0.183 | 0.215 | 0.234 | 0.280 | 0.396 | 0.583 | 0.949 | 1.160 |

*Examples 3.1-3.3: Ratio of monomer to oligomer*

*Example 3.1*

A procedure of Example 3.1 was the same as that of Example 2, and the difference was that a feed liquid contained 0.32% of $NH_4^+$ and 0.7% of $SO_4^{2-}$, and an effluent was collected when BV was equal to 1.

TOS, water, $NH_4^+$, $SO_4^{2-}$, and the pH at the outlet were monitored and summarized in Table 6.

Table 6 Composition of Example 3.1

| | TOS (%) | Water (%) | BAS (%) | AS (%) | $SO_4^{2-}$ (%) | pH | $NH_4^+$ (%) |
|---|---|---|---|---|---|---|---|
| Feed liquid entering a cation exchange resin | 89.80 | 9.82 | 0 | 1.17 | 0.70 | 0.88 | 0.32 |
| Effluent from anion exchange resin | 87.19 | 13.45 | 0 | 0.15 | 0.07 | 0.69 | 0.04 |

*Examples 3.2 and 3.3*

A sample was processed by the method of Example 3.1, and then mixed with a mixture that was not processed with ion exchange resins to obtain a mixture containing AS and BAS at a different concentration. The sample was characterized to determine an effect of the content of salts on the equilibrium between a monomer and an oligomer. Example 3.2 was a processed sample, and Example 3.3 was a sample formed by mixing the processed sample with an unprocessed solution, in which the final content of AS+BAS was 0.464%.

Results are shown in Table 7.

*Comparative Example 1: Ratio of monomer to oligomer*

Comparative Example 1 was the same as Examples 3.2 and 3.3, and the difference was that a mixture that was not processed with ion exchange resins was used.

Results are shown in Table 7.

Table 7 Ratio of monomer to oligomer

| | Comparative Example 1 | Example 3.2 | Example 3.3 |
|---|---|---|---|
| $H_2O$ (%) | 10.75 | 12.46 | 12.03 |
| AS (%) | 0.917 | 0.02 | 0.464 |
| BAS (%) | 0.534 | 0 | 0 |
| AS+BAS (%) | 1.451 | 0.02 | 0.464 |
| Monomer (M) (%) | 67.97 | 67.87 | 68.25 |
| Dimer (D) (%) | 16.09 | 16.83 | 16.47 |
| Trimer (T) (%) | 2.79 | 2.94 | 2.94 |
| M+D+T (%) | 86.85 | 87.64 | 87.66 |

It can be seen from the results, when the content of ASBAS is reduced, the content of water is increased. The sum of M+D+T is obviously affected by the content of salts, that is, as the content of salts is reduced, the content of M+D+T is increased. Under the condition of low content of salts, the sum of M+D+T can be close to 100%.

*Examples 4.1 and 4.2: Viscosity*

The samples of Examples 3.2 (Example 4.1) and 3.3 (Example 4.2) were characterized to determine an effect of the content of salts on the viscosity.

Results are shown in Table 8.

*Comparative Example 2: Viscosity*

Comparative Example 2 was the same as Examples 4.1 and 4.2, and the difference was that a mixture that was not processed with ion exchange resins was used and contained about 1.1% of AS+BAS.

Results are shown in Table 8.

Table 8 Dynamic viscosity of Example 4 and Comparative Example 2

|  | Dynamic viscosity (mPa·s) | | |
| --- | --- | --- | --- |
|  | 0°C | 10°C | 20°C |
| Example 4.1 | 278.9 | 137.4 | 104.5 |
| Example 4.2 | 333.3 | 161.8 | 120.7 |
| Comparative Example 2 | 509.7 | 386.5 | 271.2 |

*Example 5 Washins and regeneration*

After the ion exchange processing, the columns were respectively washed with 3 BV of demineralized water at a rate of 2 BV/h. The cation exchange resin was regenerated with 3 BV of 10% sulfuric acid at a rate of 2 BV/h, and the anion exchange resin was regenerated with 1.5-2 BV of 10% $NH_3 \cdot H_2O$ at a rate of 2 BV/h. 3-4 BV of demineralized water was introduced into the columns at a rate of 2 BV/h, and the mixture purified with the resins was introduced into the columns. Then, the columns could be used for the next processing cycle.

The disclosed embodiments can have various modifications and alternative forms, specific examples of which have been shown by way of examples in the drawings and are described in detail herein. However, it should be understood that present invention is only limited by the appended claims.

**Claims**

1. A mixture, comprising:

   86-89 wt% of a methionine hydroxy analog and an oligomer thereof, in which more than 99.95 wt% being monomer, dimer and trimer, and the ratio of monomer: dimer: trimer by weight being 77-80: 18.5-19.5: 3-4;
   9.2-13.5 wt% of water; and
   less than 0.5 wt% of ammonium sulfate and ammonium bisulfate;

   wherein a viscosity of the mixture at 10°C is lower than 200 mPa·s.

2. The mixture according to claim 1, wherein the ratio of monomer: dimer: trimer by weight is 77.4-78.5: 18.5-19.2: 3.2-3.6.

3. The mixture according to claim 1 or 2, wherein the viscosity of the mixture at 10°C is lower than 170 mPa s.

4. The mixture according to any one of claims 1 to 3, wherein

   the mixture comprises 9.5-12.5 wt% of water; and/or
   less than 0.4 wt% of ammonium sulfate and ammonium bisulfate.

5. The mixture according to any one of claims 1 to 4, wherein the mixture comprises less than 0.4 wt% of ammonium sulfate, and less than 0.1 wt% of ammonium bisulfate.

6. A method for processing a mixture comprising a methionine hydroxy analog and an oligomer thereof, comprising the following steps:

   S1: placing the mixture in contact with an ion exchange resin;
   S2: placing the mixture in contact with another ion exchange resin;
   S3: washing the ion exchange resin of step S1 with water to obtain an aqueous eluent, and diluting the mixture comprising a methionine hydroxy analog and an oligomer thereof with the aqueous eluent before step S1; and/or
washing the ion exchange resin of step S2 with water to obtain an aqueous eluent, and diluting the mixture comprising a methionine hydroxy analog and an oligomer thereof with the aqueous eluent before step S1; and
S4: regenerating the ion exchange resins,

preferably, steps S1 to S4 are performed on at least two groups of ion exchange resins at the same time.

7.  The method according to claim 6, wherein

step S1 comprises:
S11: placing the mixture in contact with a cation exchange resin, particularly in contact with a strong-acid cation exchange resin;
and step S2 comprises:
S21: placing the mixture in contact with an anion exchange resin, particularly in contact with a weak-base anion exchange resin;
or
step S1 comprises:
S12: placing the mixture in contact with an anion exchange resin, particularly in contact with a weak-base anion exchange resin;
and step S2 comprises:
S22: placing the mixture in contact with a cation exchange resin, particularly in contact with a strong-acid cation exchange resin.

8.  The method according to claim 6 or 7, wherein step S4 comprises:

S41: washing the cation exchange resin with an acid solution, such as a strong acid solution, particularly a sulfuric acid solution, to obtain a desorption solution, and washing the anion exchange resin with a base solution, such as a weak base solution, particularly an ammonia-water solution, to obtain another desorption solution;
S42: washing the cation exchange resin and/or anion exchange resin with water to obtain a cleaning solution; and
S43: washing the cation exchange resin and the anion exchange resin with a mixture comprising a methionine hydroxy analog and an oligomer thereof, particularly the mixture comprising a methionine hydroxy analog and an oligomer thereof that is processed at steps S1 and S2;
preferably, the desorption solutions and/or cleaning solution are circulated in the preparation process of HMTBA.

9.  The method according to claim 8, wherein

at step S41, the concentration of the sulfuric acid solution is 5-20 wt%, particularly 8-12 wt%, and the concentration of the ammonia-water solution is 2-20 wt%, particularly 4-12 wt%;
preferably, at least 2 BV, particularly 2.5-3.5 BV, of sulfuric acid solution is used, and/or at least 1.2 BV, particularly 1.5-2 BV, of ammonia-water solution is used.

10.  The method according to any one of claims 6 to 9, wherein after step S41,

the pH of an effluent from the cation exchange resin is smaller than 2, and/or the concentration of $NH_4^+$ is less than 0.1 wt%,
and/or
the pH of an effluent from the anion exchange resin is greater than 10, and/or the concentration of $SO_4^{2-}$ is less than 0.05 wt%.

11.  The method according to any one of claims 6 to 10, wherein after step S42,

the pH of the effluent from the cation exchange resin is 3-4,
and/or
the pH of the effluent from the anion exchange resin is 9-10;
and/or
at step S42, each resin is washed with at least 2 BV, particularly at least 3 BV, of water.

**12.** The method according to any one of claims 6 to 11, wherein after step S2,

the concentration of $NH_4^+$ in the mixture comprising a methionine hydroxy analog and an oligomer thereof is 0.02-0.1 wt %, and the concentration of $SO_4^{2-}$ is 0.04-0.2 wt %, and/or
the pH at 25°C is 0.3-2.5.

**13.** The method according to any one of claims 6 to 12, wherein

steps S1 and S2 are performed at 45-55°C; and/or
step S4 is performed at 40-55°C.

**14.** The method according to any one of claims 6 to 13, wherein

steps S1 and S2 are performed at a rate of 0.8-2.5 BV/h, particularly at a rate of 1-2 BV/h; and/or
step S3 is performed at a rate of 1-3 BV/h, particularly at a rate of 1.5-2.5 BV/h; and/or
step S4 is performed at a rate of 1.5-2.5 BV/h, particularly at a rate of 1.8-2.2 BV/h.

**15.** The method according to any one of claims 6 to 14, wherein

at each one of steps S1 and S2, 1 BV of ion exchange resin is used to process at most 10 BV, particularly at most 8 BV, of mixture comprising a methionine hydroxy analog and an oligomer thereof; and/or
at step S3, at least 2 BV of water is used.

**Patentansprüche**

**1.** Mischung, umfassend:

86-89 Gew.-% eines Methioninhydroxyanalogons und eines Oligomers davon, wobei mehr als 99,95 Gew.-% Monomer, Dimer und Trimer sind und das Gewichtsverhältnis von Monomer:Dimer:Trimer 77-80: 18,5-19,5: 3-4 beträgt;
9,2-13,5 Gew.-% Wasser; und
weniger als 0,5 Gew.-% Ammoniumsulfat und Ammoniumbisulfat;
wobei die Viskosität der Mischung bei 10 °C weniger als 200 mPa·s beträgt.

**2.** Mischung nach Anspruch 1, wobei das Gewichtsverhältnis von Monomer:Dimer:Trimer 77,4-78,5: 18,5-19,2: 3,2-3,6 beträgt.

**3.** Mischung nach Anspruch 1 oder 2, wobei die Viskosität der Mischung bei 10 °C weniger als 170 mPa·s beträgt.

**4.** Mischung nach einem der Ansprüche 1 bis 3, wobei die Mischung 9,5--12,5 Gew.-% Wasser umfasst; und/oder weniger als 0,4 Gew.-% Ammoniumsulfat und Ammoniumbisulfat.

**5.** Mischung nach einem der Ansprüche 1 bis 4, wobei die Mischung weniger als 0,4 Gew.-% Ammoniumsulfat und weniger als 0,1 Gew.-% Ammoniumbisulfat umfasst.

**6.** Verfahren zur Verarbeitung einer Mischung, die ein Methioninhydroxyanalogon und ein Oligomer davon umfasst, umfassend die folgenden Schritte:

S1: Inkontaktbringen der Mischung mit einem lonenaustauscherharz;
S2: Inkontaktbringen der Mischung mit einem anderen lonenaustauscherharz;
S3: Waschen des lonenaustauscherharzes aus Schritt S1 mit Wasser, um einen wässrigen Eluenten zu erhalten, und Verdünnen der Mischung, die ein Methioninhydroxyanalogon und ein Oligomer davon umfasst, mit dem wässrigen Eluenten vor Schritt S1;
und/oder
Waschen des Ionenaustauscherharzes aus Schritt S2 mit Wasser, um einen wässrigen Eluenten zu erhalten, und Verdünnen der Mischung, die ein Methioninhydroxyanalogon und ein Oligomer davon umfasst, mit dem wässrigen Eluenten vor Schritt S1; und

S4: Regenerieren der Ionenaustauscherharze,
vorzugsweise werden die Schritte S1 bis S4 an mindestens zwei Gruppen von Ionenaustauscherharzen zur gleichen Zeit durchgeführt.

7. Verfahren nach Anspruch 6, wobei Schritt S1 Folgendes umfasst:

S11: Inkontaktbringen der Mischung mit einem Kationenaustauscherharz, insbesondere mit einem stark sauren Kationenaustauscherharz;
und Schritt S2 Folgendes umfasst:

S21: Inkontaktbringen der Mischung mit einem Anionenaustauscherharz, insbesondere mit einem Anionenaustauscherharz auf schwacher Basis;
oder
Schritt S1 Folgendes umfasst:

S12: Inkontaktbringen der Mischung mit einem Anionenaustauscherharz, insbesondere mit einem Anionenaustauscherharz auf schwacher Basis;
und Schritt S2 Folgendes umfasst:
S22: Inkontaktbringen der Mischung mit einem Kationenaustauscherharz, insbesondere mit einem stark sauren Kationenaustauscherharz.

8. Verfahren nach Anspruch 6 oder 7, wobei Schritt S4 Folgendes umfasst:

S41: Waschen des Kationenaustauscherharzes mit einer sauren Lösung, wie beispielsweise einer stark sauren Lösung, insbesondere einer Schwefelsäurelösung, um eine Desorptionslösung zu erhalten, und Waschen des Anionenaustauscherharzes mit einer basischen Lösung, wie beispielsweise einer schwach basischen Lösung, insbesondere einer Ammoniakwasserlösung, um eine weitere Desorptionslösung zu erhalten;
S42: Waschen des Kationenaustauschharzes und/oder des Anionenaustauschharzes mit Wasser, um eine Reinigungslösung zu erhalten; und
S43: Waschen des Kationenaustauscherharzes und des Anionenaustauscherharzes mit einer Mischung, die ein Methioninhydroxyanalogon und ein Oligomer davon umfasst, insbesondere die Mischung, die ein Methioninhydroxyanalogon und ein Oligomer davon umfasst, das in den Schritten S1 und S2 verarbeitet wird;
vorzugsweise werden die Desorptionslösungen und/oder die Reinigungslösung im Herstellungsprozess von HMTBA zirkuliert.

9. Verfahren nach Anspruch 8, wobei in Schritt S41 die Konzentration der Schwefelsäurelösung 5-20 Gew.-%, insbesondere 8-12 Gew.-%, und die Konzentration der Ammoniakwasserlösung 2-20 Gew.-%, insbesondere 4-12 Gew.-%, beträgt;
vorzugsweise werden mindestens 2 BV, insbesondere 2,5-3,5 BV, Schwefelsäurelösung und/oder mindestens 1,2 BV, insbesondere 1,5-2 BV, Ammoniakwasserlösung verwendet.

10. Verfahren gemäß einem der Ansprüche 6 bis 9, wobei nach dem Schritt S41,

der pH-Wert eines Ausflusses aus dem Kationenaustauscherharz kleiner als 2 ist und/oder die Konzentration von $NH_4^+$ weniger als 0,1 Gew.-% beträgt,
und/oder
der pH-Wert eines Ausflusses aus dem Anionenaustauscherharz größer als 10 ist und/oder die Konzentration von $SO_4^{2-}$ weniger als 0,05 Gew.-% beträgt.

11. Verfahren nach einem der Ansprüche 6 bis 10, wobei nach Schritt S42 der pH-Wert des Abflusses aus dem Kationenaustauscherharz 3-4 beträgt,
und/oder

der pH-Wert des Ausflusses aus dem Anionenaustauscherharz 9-10 beträgt;
und/oder
in Schritt S42 jedes Harz mit mindestens 2 BV, insbesondere mindestens 3 BV, Wasser gewaschen wird.

12. Verfahren gemäß einem der Ansprüche 6 bis 11, wobei nach dem Schritt S2,

die Konzentration von NH$_4^+$ in der Mischung, die ein Methioninhydroxyanalogon und ein Oligomer davon umfasst, 0,02-0,1 Gew.-% und die Konzentration von SO$_4^{2-}$ 0,04-0,2 Gew.-% beträgt, und/oder
der pH-Wert bei 25 °C 0,3-2,5 beträgt.

**13.** Verfahren nach einem der Ansprüche 6 bis 12, wobei

die Schritte S1 und S2 bei 45-55 °C durchgeführt werden; und/oder
Schritt S4 bei 40-55 °C durchgeführt wird.

**14.** Verfahren nach einem der Ansprüche 6 bis 13, wobei

die Schritte S1 und S2 mit einer Geschwindigkeit von 0,8-2,5 BV/h, insbesondere mit einer Geschwindigkeit von 1-2 BV/h, durchgeführt werden; und/oder
Schritt S3 mit einer Geschwindigkeit von 1-3 BV/h, insbesondere mit einer Geschwindigkeit von 1,5-2,5 BV/h durchgeführt wird; und/oder
Schritt S4 mit einer Geschwindigkeit von 1,5-2,5 BV/h durchgeführt wird, insbesondere mit einer Geschwindigkeit von 1,8-2,2 BV/h.

**15.** Verfahren nach einem der Ansprüche 6 bis 14, wobei

in jedem der Schritte S1 und S2 1 BV Ionenaustauscherharz verwendet wird, um höchstens 10 BV, insbesondere höchstens 8 BV, einer Mischung zu verarbeiten, die ein Methioninhydroxyanalog und ein Oligomer davon umfasst; und/oder
in Schritt S3 mindestens 2 BV Wasser verwendet wird.

## Revendications

**1.** Mélange, comprenant :

86 à 89% en poids d'un analogue hydroxy de la méthionine et d'un oligomère de celui-ci, dans lesquels plus de 99,95% en poids est un monomère, dimère et trimère, le rapport monomère : dimère : trimère en poids étant de 77-80 : 18,5-19,5 : 3-4 ;
9,2-13,5% en poids d'eau ; et
moins de 0,5% en poids de sulfate d'ammonium et de bisulfate d'ammonium ;
dans lequel une viscosité du mélange à 10°C est inférieure à 200 mPas.

**2.** Mélange selon la revendication 1, dans lequel le rapport monomère : dimère : trimère en poids est de 77,4-78,5 : 18,5-19,2 : 3,2-3,6.

**3.** Mélange selon la revendication 1 ou 2, dans lequel la viscosité du mélange à 10°C est inférieure à 170 mPas.

**4.** Mélange selon l'une quelconque des revendications 1 à 3, dans lequel

le mélange comprend 9,5-12,5% en poids d'eau ; et/ou
moins de 0,4% en poids de sulfate d'ammonium et de bisulfate d'ammonium.

**5.** Mélange selon l'une quelconque des revendications 1 à 4, dans lequel le mélange comprend moins de 0,4% en poids de sulfate d'ammonium et moins de 0,1% en poids de bisulfate d'ammonium.

**6.** Procédé de traitement d'un mélange comprenant un analogue hydroxy de la méthionine et un oligomère de celui-ci, comprenant les étapes suivantes :

S1 : mise en contact du mélange avec une résine échangeuse d'ions ;
S2 : mise en contact du mélange avec une autre résine échangeuse d'ions ;
S3 : lavage de la résine échangeuse d'ions de l'étape S1 avec de l'eau pour obtenir un éluant aqueux, et dilution du mélange comprenant un analogue hydroxy de la méthionine et un oligomère de celui-ci avec l'éluant aqueux avant l'étape S1 ;

et/ou
lavage de la résine échangeuse d'ions de l'étape S2 avec de l'eau pour obtenir un éluant aqueux, et dilution du mélange comprenant un analogue hydroxy de la méthionine et un oligomère de celui-ci avec l'éluant aqueux avant l'étape S1 ; et
S4 : régénération des résines échangeuses d'ions,
de préférence, les étapes S1 à S4 sont réalisées sur au moins deux groupes de résines échangeuses d'ions en même temps.

7. Procédé selon la revendication 6, dans lequel

l'étape S1 comprend :
S11 : mise en contact du mélange avec une résine échangeuse de cations, en particulier avec une résine échangeuse de cations fortement acide ; et
l'étape S2 comprend :
S21 : mise en contact du mélange avec une résine échangeuse d'anions, en particulier avec une résine échangeuse d'anions faiblement basique ;
ou
l'étape S1 comprend :
S12 : mise en contact du mélange avec une résine échangeuse d'anions, en particulier avec une résine échangeuse d'anions faiblement basique ;
et l'étape S2 comprend :
S22 : mise en contact du mélange avec une résine échangeuse de cations, en particulier avec une résine échangeuse de cations fortement acide.

8. Procédé selon la revendication 6 ou 7, dans lequel l'étape S4 comprend :

S41 : lavage de la résine échangeuse de cations avec une solution acide, telle qu'une solution d'acide fort, en particulier une solution d'acide sulfurique, pour obtenir une solution de désorption, et lavage de la résine échangeuse d'anions avec une solution basique, telle qu'une solution de base faible, en particulier une solution ammoniac-eau, pour obtenir une autre solution de désorption ;
S42 : lavage de la résine échangeuse de cations et/ou de la résine échangeuse d'anions avec de l'eau pour obtenir une solution de nettoyage ; et
S43: lavage de la résine échangeuse de cations et de la résine échangeuse d'anions avec un mélange comprenant un analogue hydroxy de la méthionine et un oligomère de celui-ci, en particulier le mélange comprenant un analogue hydroxy de la méthionine et un oligomère de celui-ci qui est traité aux étapes S1 et S2 ;
de préférence, les solutions de désorption et/ou la solution de nettoyage circulent dans le processus de préparation de HMTBA.

9. Procédé selon la revendication 8, dans lequel

à l'étape S41, la concentration de la solution d'acide sulfurique est de 5-20% en poids, en particulier de 8-12% en poids, et la concentration de la solution ammoniac-eau est de 2-20% en poids, en particulier de 4-12% en poids ;
de préférence, au moins 2 BV, en particulier 2,5-3,5 BV, de solution d'acide sulfurique sont utilisés, et/ou au moins 1,2 BV, en particulier 1,5-2 BV, de solution ammoniac-eau sont utilisés.

10. Procédé selon l'une quelconque des revendications 6 à 9, dans lequel après l'étape S41,

le pH d'un effluent provenant de la résine échangeuse de cations est inférieur à 2, et/ou la concentration en $NH_4^+$ est inférieure à 0,1% en poids,
et/ou
le pH d'un effluent provenant de la résine échangeuse d'anions est supérieur à 10, et/ou la concentration en $SO_4^{2-}$ est inférieure à 0,05% en poids.

11. Procédé selon l'une quelconque des revendications 6 à 10, dans lequel après l'étape S42,

le pH de l'effluent provenant de la résine échangeuse de cations est de 3-4,
et/ou

le pH de l'effluent provenant de la résine échangeuse d'anions est de 9-10 ;
et/ou
à l'étape S42, chaque résine est lavée avec au moins 2 BV, en particulier au moins 3 BV, d'eau.

**12.** Procédé selon l'une quelconque des revendications 6 à 11, dans lequel après l'étape S2,

la concentration en $NH_4^+$ dans le mélange comprenant un analogue hydroxy de la méthionine et un oligomère de celui-ci est de 0,02-0,1% en poids et la concentration en $SO_4^{2-}$ est de 0,04-0,2% en poids, et/ou
le pH à 25°C est de 0,3-2,5.

**13.** Procédé selon l'une quelconque des revendications 6 à 12, dans lequel

les étapes S1 et S2 sont réalisées à une température de 45-55°C ; et/ou
l'étape S4 est réalisée à une température de 40-55°C.

**14.** Procédé selon l'une quelconque des revendications 6 à 13, dans lequel

les étapes S1 et S2 sont réalisées à un débit de 0,8-2,5 BV/h, en particulier à un débit de 1-2 BV/h ; et/ou
l'étape S3 est réalisée à un débit de 1-3 BV/h, en particulier à un débit de 1,5-2,5 BV/h ; et/ou
l'étape S4 est réalisée à un débit de 1,5-2,5 BV/h, en particulier à un débit de 1,8-2,2 BV/h.

**15.** Procédé selon l'une quelconque des revendications 6 à 14, dans lequel

à chacune des étapes S1 et S2, 1 BV de résine échangeuse d'ions est utilisé pour traiter au plus 10 BV, en particulier au plus 8 BV, de mélange comprenant un analogue hydroxy de la méthionine et un oligomère de celui-ci ; et/ou
à l'étape S3, au moins 2 BV d'eau sont utilisés.

S1: placing mixture in contact with ion exchange resin

S2: placing the mixture in contact with another ion exchange resin

S3: washing the ion exchange resins by using water

S4: regenerating the ion exchange resins

Fig. 1

AS solution

MMP
ammonia → cyanohydrin
natural gas → preparation unit
sulfuric acid

HMTBN

H₂SO₄ → HMTBA preparation unit

AS solution → AS preparation unit

pure solid AS

desorption solution / cleaning solution obtained by the resin processing

HBTBA product

desorption solution / cleaning solution obtained by the resin processing

water

resin processing unit

HMTBA product with low content of salts

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 109232336 A
- CN 1186068 A